# EUROPEAN PATENT APPLICATION

(11) **EP 4 834 389 A1**
(43) Date of publication of application: **04.11.2026**
(21) Application number: 26175959.1
(22) Date of filing: 30.04.2026
(51) Int. Cl.: A61M 3/02

(54) **NASAL SPRAY TIP**

(30) Priority: 30.04.2025 US 202519195055
(71) Applicant: FridaBaby, LLC, Miami, FL 33137 (US)
(72) Inventor: HIRSCHHORN, Chelsea, Miami, FL 33137 (US); FORNARO, Sophie, Miami, FL 33137 (US); CACCIOLA, Chris, Miami, FL 33137 (US); VILLALOBOS, Janette, Miami, FL 33137 (US); HUNTER, Jenna, Miami, FL 33137 (US)
(74) Representative: Lissandrini, Marco

(57) **Abstract**

An apparatus for nasal rinsing includes a pressurized canister comprising a solution and configured to deliver the solution to a user. The apparatus also includes an actuator operably coupled to the pressurized canister and configured to dispense the solution in a non-atomized fluid stream upon actuation. The apparatus further includes an actuator tip formed being removably coupled to a nozzle of the actuator. The actuator tip includes a first retention feature that engages a second retention feature of the nozzle to form a snap-fit that retains the actuator tip to the nozzle during use.

## Description

### FIELD OF THE DISCLOSURE

Aspects of the present disclosure generally relate to nasal rinse apparatus, and more specifically to a nasal rinse apparatus with a removable actuator tip.

### BACKGROUND

Nasal irrigation, also referred to as nasal rinsing or nasal lavage, is a common technique used to relieve nasal congestion and remove mucus, allergens, and other debris from the nasal passages. Such rinsing is often accomplished by delivering a fluid, such as a saline solution, into one nostril and allowing it to flow through the nasal cavity and exit through the opposite nostril. This process can be particularly helpful in alleviating symptoms associated with colds, allergies, or sinus infections.

### SUMMARY

Various aspects of the present disclosure are directed to an apparatus for nasal rinsing. The apparatus includes a pressurized canister comprising a solution and configured to deliver the solution to a user. The apparatus also includes an actuator operably coupled to the pressurized canister and configured to dispense the solution in a non-atomized fluid stream upon actuation. The apparatus further includes an actuator tip formed being removably coupled to a nozzle of the actuator. The actuator tip includes a first retention feature that engages a second retention feature of the nozzle to form a snap-fit that retains the actuator tip to the nozzle during use.

Some aspects of the present disclosure are directed to method of performing nasal irrigation using a pressurized nasal rinsing device. The method includes attaching an actuator tip to a nozzle of an actuator using a snap-fit engagement. The actuator tip includes a first retention feature that engages a second retention feature of the nozzle to retain the actuator tip to the nozzle during use via the snap-fit engagement. The method also includes positioning the actuator tip against a user's nostril such that the tip forms a seal and limits insertion depth. The method further includes actuating the nasal rinsing device to deliver a solution through the actuator tip in a non-atomized fluid stream. The method still further includes removing the actuator tip following use.

The foregoing has outlined rather broadly the features and technical advantages of examples according to the disclosure in order that the detailed description that follows may be better understood. Additional features and advantages will be described. The conception and specific examples disclosed may be readily utilized as a basis for modifying or designing other structures for carrying out the same purposes of the present disclosure. Such equivalent constructions do not depart from the scope of the appended claims. Characteristics of the concepts disclosed, both their organization and method of operation, together with associated advantages will be better understood from the following description when considered in connection with the accompanying figures. Each of the figures is provided for the purposes of illustration and description, and not as a definition of the limits of the claims.

### BRIEF DESCRIPTION OF THE DRAWINGS

So that the above-recited features of the present disclosure can be understood in detail, a more particular description may be had by reference to aspects, some of which are illustrated in the appended drawings. It is to be noted, however, that the appended drawings illustrate only certain typical aspects of this disclosure and are therefore not to be considered limiting of its scope, for the description may admit to other equally effective aspects. The same reference numbers in different drawings may identify the same or similar elements.
Figure 1A is a diagram of a nasal spray device, in accordance with various aspects of the present disclosure.
Figure 1B is a diagram illustrating an example cross-sectional view of the nasal spray device, in accordance with various aspects of the present disclosure.
Figure 1C is a diagram illustrating an example of a nasal spray device, in accordance with various aspects of the present disclosure.
Figures 2A, 2B, 2C, and 2D illustrate various views and examples of an actuator tip, in accordance with aspects of the present disclosure.
Figures 3A, 3B, and 3C illustrate various views of another example of an actuator tip, in accordance with various aspects of the present disclosure.
Figures 4A, 4B, and 4C illustrate another example of an actuator tip for use with a nasal rinsing device, in accordance with various aspects of the present disclosure.
Figure 5 is a is a flow diagram illustrating a process of using a nasal spray device, in accordance with various aspects of the present disclosure.

### DETAILED DESCRIPTION

The detailed description set forth below, in connection with the appended drawings, is intended as a description of various configurations and is not intended to represent the only configurations in which the concepts described herein may be practiced. The detailed description includes specific details for the purpose of providing a thorough understanding of the various concepts. It will be apparent to those skilled in the art, however, that these concepts may be practiced without these specific details. In some instances, well-known structures and components are shown in block diagram form in order to avoid obscuring such concepts.

Based on the teachings, one skilled in the art should appreciate that the scope of the present disclosure is intended to cover any aspect of the present disclosure, whether implemented independently of or combined with any other aspect of the present disclosure. For example, an apparatus may be implemented, or a method may be practiced using any number of the aspects set forth. In addition, the scope of the present disclosure is intended to cover such an apparatus or method practiced using other structure, functionality, or structure and functionality in addition to, or other than the various aspects of the present disclosure set forth. It should be understood that any aspect of the present disclosure may be embodied by one or more elements of a claim.

The word "exemplary" is used herein to mean "serving as an example, instance, or illustration." Any aspect described herein as "exemplary" is not necessarily to be construed as preferred or advantageous over other aspects.

Although particular aspects are described herein, many variations and permutations of these aspects fall within the scope of the present disclosure. Although some benefits and advantages of the preferred aspects are mentioned, the scope of the present disclosure is not intended to be limited to particular benefits, uses, or objectives. Rather, aspects of the present disclosure are intended to be broadly applicable to different technologies, system configurations, networks, and protocols, some of which are illustrated by way of example in the figures and in the following description of the preferred aspects. The detailed description and drawings are merely illustrative of the present disclosure rather than limiting, the scope of the present disclosure being defined by the appended claims and equivalents thereof.

Nasal irrigation, also referred to as nasal rinsing or nasal lavage, is a common technique used to relieve nasal congestion and remove mucus, allergens, and other debris from the nasal passages. Such rinsing is often accomplished by delivering a fluid, such as a saline solution, into one nostril and allowing it to flow through the nasal cavity and exit through the opposite nostril. This process can be particularly helpful in alleviating symptoms associated with colds, allergies, or sinus infections.

Conventional nasal rinsing devices include manual squeeze bottles, bulb syringes, or gravity-based systems. While generally effective at clearing nasal passages, these conventional devices can present challenges, particularly for young children or individuals with limited dexterity. For example, traditional squeeze bottles may require two-handed operation, precise positioning, and user-applied pressure to achieve adequate flow. Moreover, existing devices may lack safeguards that prevent excessive insertion depth, which could cause discomfort or harm to the user, especially in pediatric applications. Some powered or pressurized solutions have been proposed to address these issues, but these systems often involve complex delivery mechanisms, motors, and electronics. Additionally, conventional systems often fail to provide a comfortable interface for pediatric use and may lack secure, hygienic, and user-removable mechanisms for attaching a nasal tip to the fluid delivery system.

Accordingly, it may be desirable to improve a nasal rinsing device to provide a controlled, pressurized fluid delivery system suitable for children as young as two years old, while also offering a user-friendly, safe, and comfortable interface. Various aspects of the present disclosure are directed to a nasal rinsing apparatus (e.g., nasal rinsing device) for delivering a pressurized saline solution safely and comfortably, particularly for pediatric users aged two years and older. In some examples, the device provides a one-handed, user-friendly solution that simplifies nasal irrigation while improving safety, hygiene, and comfort over conventional systems. In some such examples, the device includes a pressurized canister containing a saline solution and configured to deliver the solution at a controlled pressure, such as, for example, between approximately 82.737,1 Pa and 96.526,6 Pa (i.e., approximately 12 and 14 psi) and a flowrate of approximately 1.6 mL/s. This controlled delivery enables effective nasal lavage while reducing the risk of excessive force or insufficient rinsing-issues commonly associated with manual squeeze bottles or syringes, which rely on inconsistent user-applied pressure.

In some implementations, an actuator, such as a rigid actuator, may be operably coupled to the canister and configured to dispense a solution, such as a saline solution, in a continuous, non-atomized fluid stream upon actuation. A soft actuator tip may be attached to the actuator. The actuator tip may be made of a flexible silicone material. The actuator tip may be specified for sealing engagement with the user's nostril, and its geometry may be configured to limit a depth of insertion to improve safety for young children. Without an effective seal, in cases of high congestion the pressurized liquid cannot travel through the nasal cavity and could return out of the same nostril resulting in an ineffective rinse. In some examples, the actuator may include a hard plastic body with an annular snap ring that mates with a corresponding annular recess in the actuator tip, forming a secure yet user-removable snap-fit connection. This design ensures that the tip remains attached during use while still allowing for hygienic removal and replacement. Importantly, the inner wall of the actuator tip is continuous and smooth, free of sharp transitions or step interfaces that could cause discomfort, even while accommodating the mechanical snap joint. These features collectively provide a nasal rinsing solution that overcomes key drawbacks of conventional systems, such as discomfort, inconsistent pressure delivery, poor sealing, or risk of tip dislodgement. By pairing a rigid actuator body with a soft, ergonomically shaped tip, the disclosed apparatus achieves an optimal balance of structural integrity, safety, and user comfort.

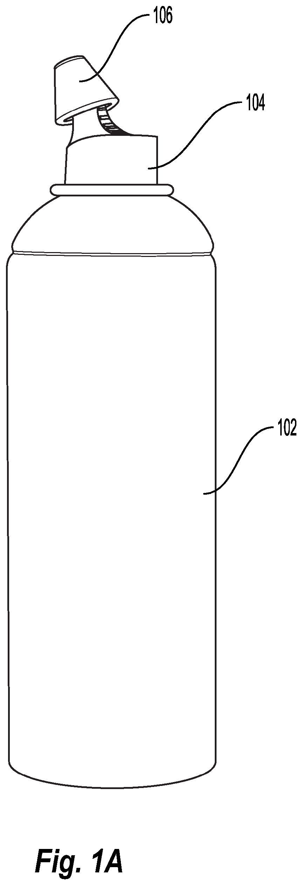

Figure 1A is a diagram of a nasal spray device 100, in accordance with various aspects of the present disclosure. As shown in the example of Figure 1A, the nasal spray device 100 includes a solution can 102, an actuator 104, and a soft actuator tip 106. These components are configured to collectively deliver a solution, such as a saline rinse, in a controlled, pressurized fluid stream for nasal lavage. The solution can 102 is an example of a pressurized container that holds a solution, such as a sterile saline solution. In one example, the solution can 102 and the actuator 104 are configured to deliver the saline solution at a pressure between approximately 82.737,1 Pa and 96.526,6 Pa (i.e., approximately 12 and 14 pounds per square inch (psi)), with a flowrate of approximately 1.6 milliliters per second (mL/s). This pressure and flowrate may provide effective nasal rinsing while maintaining safety and comfort for pediatric users, such as users as young as two years old. The can may be formed of aluminum, steel, or another suitable material compatible with medical-grade saline and propellant mixtures.

The actuator 104 may be mounted atop the solution can 102 and configured to control the release of the saline solution. In some examples, the actuator 104 includes a valve assembly that is engaged when a downward force is applied, allowing the pressurized solution to flow through the actuator 104 and out of the actuator tip 106. The actuator 104 may be made of a rigid thermoplastic or other durable, moldable polymer and is shaped to allow for one-handed operation by the user. The actuator tip 106 is coupled to the distal end of actuator 104 and serves as the nasal interface. In some examples, the actuator tip 106 is formed from a soft, flexible silicone material, allowing it to comfortably seal against the inner rim of the user's nostril. The actuator tip 106 may have a specific geometry to limit the depth of insertion into the nostril, reducing the risk of discomfort or injury, particularly in pediatric applications. In some examples, the actuator tip 106 may include an alignment tab or guide structure that ensures correct angular orientation when coupled to actuator 104. This may be particularly useful where the tip geometry includes asymmetrical sealing surfaces or flow channels.

In some examples, the actuator tip 106 is removably coupled to actuator 104 via a mechanical snap-fit connection. Specifically, the actuator 104 may include an annular snap ring, while actuator tip 106 includes a corresponding annular recess. This interface creates a secure connection that resists detachment during use, yet allows for manual removal and replacement of the actuator tip 106 for cleaning or hygienic disposal. In some examples, the internal wall of actuator tip 106 may form a continuous, smooth surface that transitions seamlessly into the actuator 104. This interior design may eliminate abrupt changes in geometry or hard edges that might otherwise be felt within the user's nostril. In one example, the actuator tip is a single-wall construction that maintains surface continuity despite the presence of the snap-fit features embedded within its outer wall.

In some examples, the actuator 104 may be integrated directly with the can 102 as a unitary structure, rather than being separately attachable. Additionally, or alternatively, the actuator 104 may include an extended lever or button to assist users with limited dexterity or motor control. Additionally, or alternatively, while actuator tip 106 may be made of soft silicone, other biocompatible elastomeric materials may be used, such as thermoplastic elastomers (TPE) or medical-grade rubber. Additionally, or alternatively, the geometry of the tip 106 may also vary, including conical, flared, or oval-shaped designs, provided that insertion depth remains limited and comfort is maintained.

Although the described embodiment is configured for pressurized delivery of a saline solution, aspects of the present disclosure are not limited to the saline solution. Other fluids, such as medicated nasal rinses or buffered solutions, may be used. The pressure and flowrate may be varied depending on the intended age range or clinical indication.

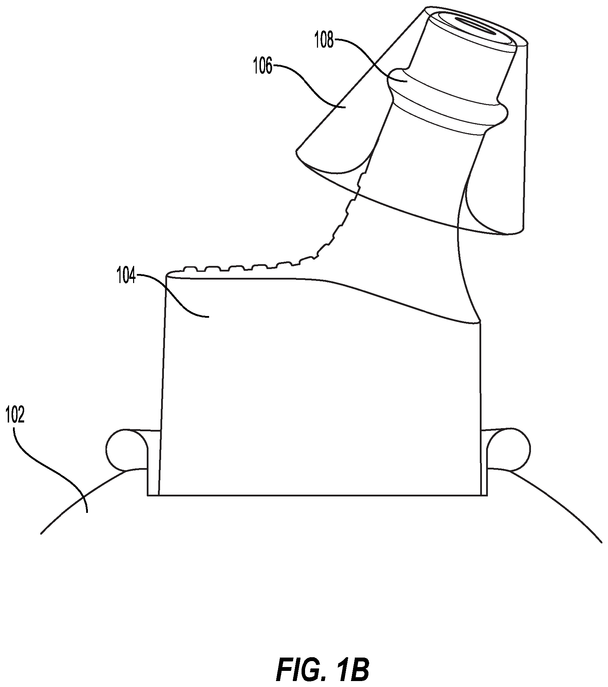

Figure 1B is a diagram illustrating an example cross-sectional view of the nasal spray device 100, in accordance with various aspects of the present disclosure. As shown, nasal spray device 100 includes a solution can 102, an actuator 104, and a soft actuator tip 106. As shown in the example of Figure 1B, the solution can 102 includes an upper valve region that mates with the lower end of actuator 104. The actuator 104 is mounted to the pressurized solution can 102 and is configured to permit release of the saline solution upon application of a downward force. The actuator 104 includes an internal channel or valve stem that directs the flow of fluid from the can 102 upward into actuator tip 106. Alternatively, the actuator 104 may be attached to a valve stem of the can 102. Additionally, the actuator tip 106 is removably coupled to a nozzle 112 of the actuator 104. The nozzle 112 may extend outward, at an angle, from a body of the actuator 104. In some examples, the actuator tip 106 is formed from a soft, elastomeric material, such as medical-grade silicone, and includes a generally conical or tapered shape to allow for sealing engagement with a user's nostril.

As shown in the example of Figure 1B, the actuator 104 may be connected to the actuator tip 106 via a snap-fit retention mechanism. Specifically, as shown in Figure 1B, the actuator 104 includes a snap ring 108, which is an annular ridge formed on an outer surface of the nozzle 112. The inner surface of actuator tip 106 includes a complementary annular recess configured to receive the snap ring 108. The engagement between snap ring 108 and the annular recess in actuator tip 106 provides a mechanical interference fit that securely retains the actuator tip 106 during normal operation, including during pressurized dispensing of the solution from the can 102. The snap-fit withstands axial and radial forces experienced during use, while still permitting manual removal of the actuator tip 106 by the user for cleaning or replacement. Notably, despite the inclusion of snap ring 108, the interior surface of actuator tip 106 remains smooth and continuous in the region inserted into the user's nostril. The snap engagement is located outside of the fluid channel and does not protrude into the internal wall of the actuator tip. This arrangement ensures that the user experiences a comfortable, seamless interface during nasal insertion, without bumps or hard transitions that could otherwise cause discomfort or injury.

The snap ring 108 may be formed integrally with the actuator 104 or added as a separate ring component during assembly. The annular geometry of the snap-fit may also be replaced in other embodiments with non-annular engagement features such as keyed tabs or bayonet-style flanges. The materials, dimensions, and tolerances of snap ring 108 and the corresponding recess may be selected to optimize retention force and user removability. For example, in some implementations, the interference may be configured to provide a pull-off force between approximately 3.5 - 4.0 pound force which is sufficient to prevent accidental disconnection while allowing for intentional removal.

As used herein, the term "optimized" refers to a configuration in which the materials, dimensions, and tolerances of the snap ring 108 and the corresponding recess are selected to achieve a functional balance between two competing objectives: providing sufficient retention force to prevent unintentional disconnection of the actuator tip 106 during normal use, including during pressurized fluid dispensing, and allowing for intentional removal of the actuator tip 106 by the user without excessive force or risk of damage to the components. In particular, optimization may include adjusting the interference fit, material hardness, and geometry of the snap ring and recess to produce a pull-off force within a target range. A pull-off force below this range may result in insecure attachment, while a force above this range may cause difficulty in removal or deformation of the actuator tip 106. Optimization may also involve consideration of manufacturing tolerances and material memory, particularly for elastomeric materials, such as silicone, to ensure consistent engagement and disengagement performance over repeated use cycles.

A shape of the actuator tip 106 is not limited to the shape shown in the examples of Figures 1A and 1B, other shapes are contemplated.

In some examples, a distal end of the nozzle 112 and the interior flow channel of the actuator tip 106 may be configured such that their respective outlet surfaces are substantially coplanar. As used herein, the term coplanar refers to a configuration in which the outlet opening of the nozzle 112 terminates in the same geometric plane as the adjacent interior surface of the actuator tip 120, resulting in a continuous and uninterrupted fluid pathway through the actuator tip 106. In such configurations, the actuator tip 106 is molded or formed to conform precisely around the exit portion of nozzle 112, such that no internal step, lip, or discontinuity is present between the fluid discharge point of the nozzle and the inner lumen of the actuator tip. This may be achieved by ensuring that the inner diameter of the actuator tip matches the outer diameter of the nozzle exit, and that both terminate in a shared horizontal plane perpendicular to the fluid flow axis. This coplanar arrangement offers several functional benefits. First, it helps ensure a smooth and laminar flow of the saline solution through the actuator tip and into the user's nostril, reducing turbulence and improving comfort. Second, it eliminates sharp or abrupt transitions inside the nasal tip, which may otherwise be felt by the user during insertion or could potentially trap residual fluid or debris. Third, it enables more precise directional control of the rinse stream, which may be important for efficient nasal cavity cleansing and for minimizing user discomfort or splashback.

As used herein, the term lumen refers to the interior passage or channel within a tubular structure through which fluid may flow. In the context of the present disclosure, the lumen of the actuator tip defines the internal conduit that extends from the actuator or nozzle to the distal outlet of the tip. This lumen provides a pathway for the saline solution, via the nozzle, as it is dispensed from the pressurized canister and delivered into the user's nasal cavity. The lumen may be enclosed by the inner wall of the actuator tip and may be cylindrical, conical, or tapered in shape.

In some examples, the nozzle 112 may include a slightly beveled or flared terminal edge that blends seamlessly into the inner surface of the actuator tip 106. In another example, the tip 106 may be molded directly over the nozzle exit in a co-molding or insert molding process to ensure exact coplanarity. In a friction-fit embodiment, the actuator tip 106 may be dimensioned such that its axial insertion onto the nozzle 112 is precisely controlled to position the outlet of the nozzle substantially flush with the interior surface of the tip, thereby achieving or closely approximating a coplanar fluid exit.

In some other examples, the nozzle 112 and actuator tip 106 may terminate in surfaces that are not perfectly coplanar but are configured such that the offset or gap is less than a threshold amount, for example, less than 0.7 mm. In such examples, the minor variation is still sufficient to preserve a smooth, non-irritating interface for user contact.

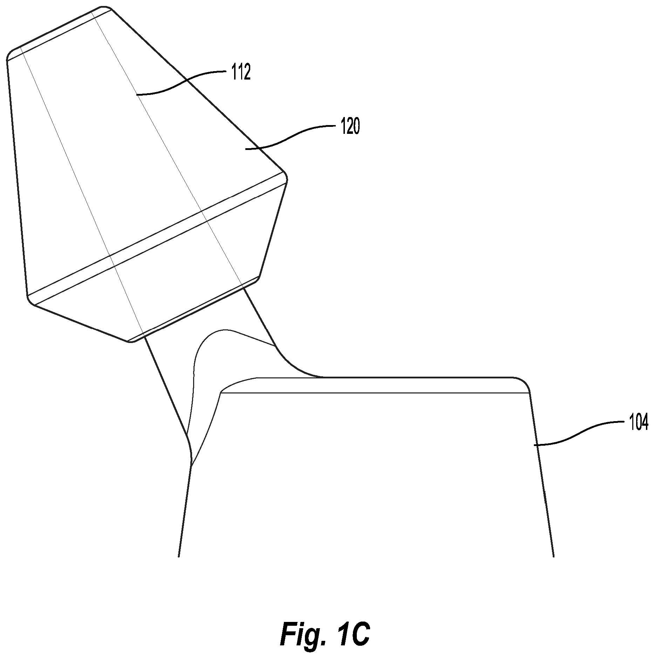

Figure 1C is a diagram illustrating an example of a nasal spray device 100, in accordance with various aspects of the present disclosure. In the example of Figure 1C, an is coupled to the actuator 104 via a friction fit, rather than a mechanical snap-fit or interlocking structure. The actuator tip 120 has a different shape than the actuator tip 106 described with reference to Figures 1A and 1B, however, the friction fit is not limited to the specific type of actuator tip 120 shown in Figure 1C, other shapes are contemplated.

As shown in the example of Figure 1C, the actuator 104 includes a nozzle 112 that is positioned within actuator tip 120 and directs the saline solution outward in a continuous, non-atomized fluid stream when the actuator is engaged. The nozzle 112 may be integrally formed with actuator 104 or may be a separate component coupled within the actuator body. In the example of Figure 1C, the actuator tip 120 is positioned over the nozzle 112 and retained by radial interference between the inner surface of the actuator tip and the outer surface of the nozzle or adjacent actuator body. The friction fit between the actuator tip 120 and the nozzle 112 provides sufficient retention force during normal use, while still allowing the tip to be removed by the user for cleaning or replacement. In this example, no annular snap ring or mechanical locking feature is present; instead, retention is achieved through precise dimensional tolerances and material compliance.

The actuator tip 120 may be formed from a soft, elastomeric material, such as silicone or thermoplastic elastomer (TPE), which allows the tip to flex and conform to the mating surface while maintaining an effective seal. As with the snap-fit embodiment, the geometry of tip 120 may include a conical or tapered insertion section to comfortably engage a user's nostril and limit insertion depth. The friction-fit configuration shown in the example of Figure 1C may offer advantages in terms of ease of manufacturing, reduced part complexity, and compatibility with certain actuator designs where a mechanical interlock is not feasible. In some embodiments, the friction fit may be supplemented by a circumferential ridge or shallow recess to improve alignment and reduce the likelihood of unintentional disconnection.

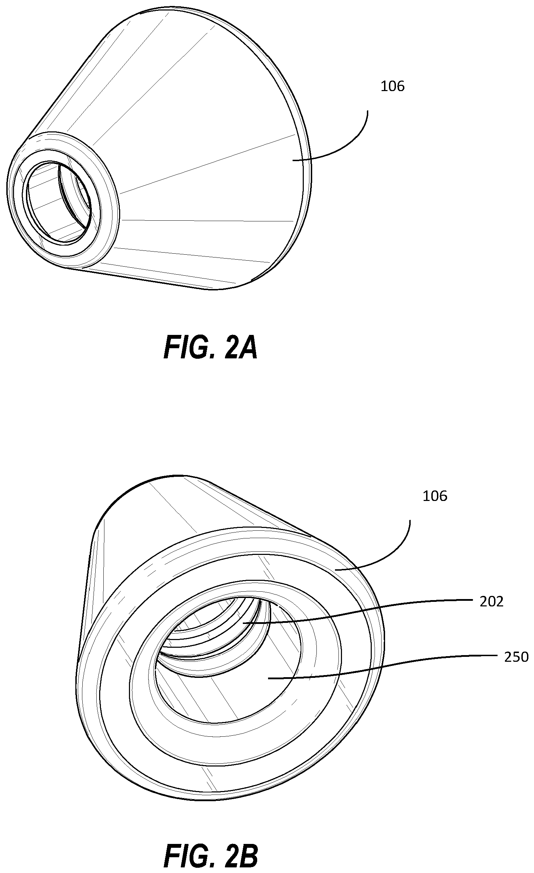

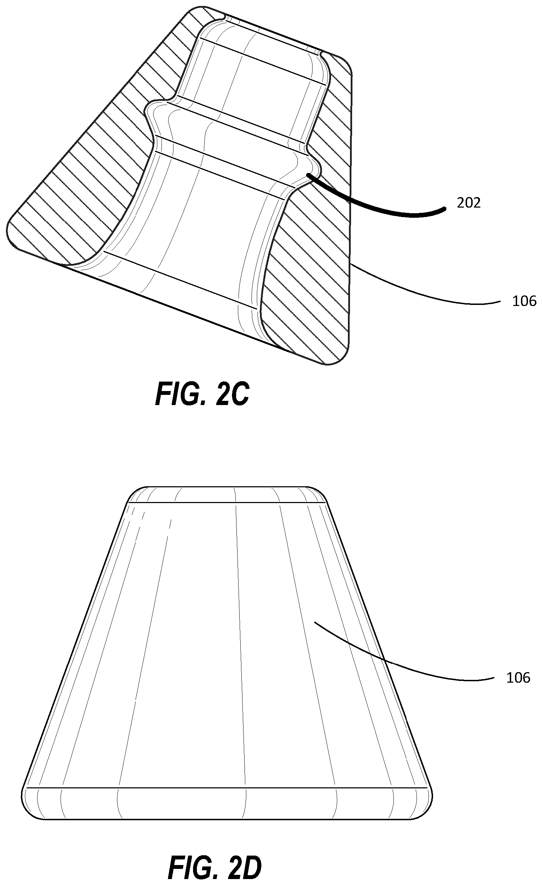

Figures 2A, 2B, 2C, and 2D illustrate various views and examples of an actuator tip 106, in accordance with aspects of the present disclosure. The actuator tip 106 is designed to be coupled to a rigid actuator and to interface comfortably with the user's nostril during nasal rinsing.

Figure 2A illustrates a front perspective view of the actuator tip 106, in accordance with various aspects of the present disclosure. The tip 106 includes a generally conical or frustoconical outer body configured for insertion at the opening of a user's nostril. The distal end (inward-facing) of the actuator tip defines a central lumen that allows for fluid communication between the actuator and the user. The overall geometry of the tip 106 enables gentle nasal sealing while preventing over-insertion, particularly for pediatric users.

Figure 2B is a diagram illustrating a rear view of actuator tip 106, in accordance with various aspects of the present disclosure. Specifically, as shown in Figure 2B, an annular ring 202 is disposed on the interior surface 250 of the tip 106. The annular ring 202 is configured to engage with a corresponding annular groove or snap ridge on a nozzle, forming a mechanical snap-fit connection that retains the actuator tip 106 in position during use. The ring 202 may be integrally molded with the actuator tip 106 and formed from the same soft elastomeric material. The ring's radial dimensions and material compliance are optimized to balance retention force with ease of removal.

Figure 2C is a diagram illustrating an example of a cross-sectional view of the actuator tip 106, in accordance with various aspects of the present disclosure. As shown in the example of Figure 2C, the annular ring 202 protrudes radially inward from the inner wall of actuator tip 106, forming a recess or cavity. This structure creates a radial interference zone when mated with a corresponding feature on the nozzle, ensuring a secure connection during fluid dispensing. The cross-section also reveals that the actuator tip 106 may be of single-wall construction, maintaining a smooth, uninterrupted inner surface for user comfort.

Figure 2D illustrates an example of a side view of actuator tip 106, in accordance with various aspects of the present disclosure. As shown in the example of Figure 2D, the outer surface is rounded and may feature a smooth or lightly textured surface to aid with sealing and comfort. The opening through the tip 106 is dimensioned to allow unobstructed passage of a fluid stream, such as a saline solution, and may be configured to maintain a coplanar or near-coplanar interface with the nozzle exit of the actuator to minimize internal transitions or turbulence.

The actuator tip 106 may be formed from biocompatible, medical-grade silicone or other flexible elastomeric materials. In some examples, the annular ring 202 may be shaped with one or more detents, undercuts, or variable cross-sections to modify the retention force profile. The tip may be disposable, reusable, or color-coded for different age groups or pressure settings.

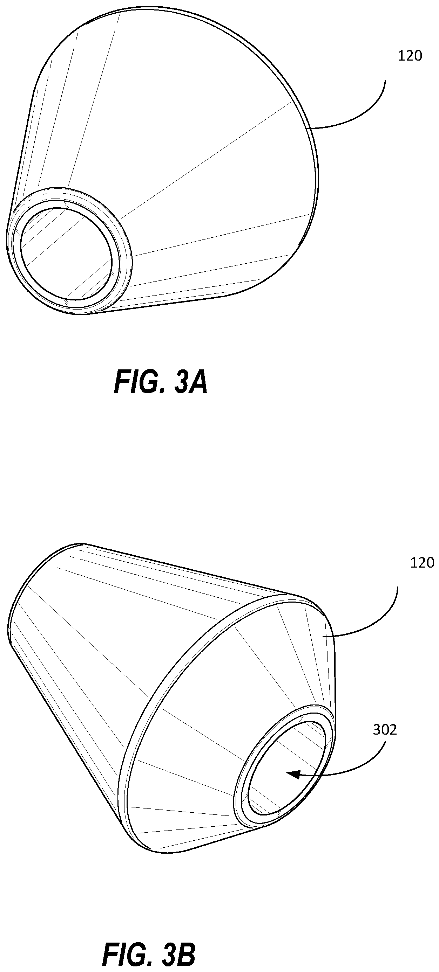

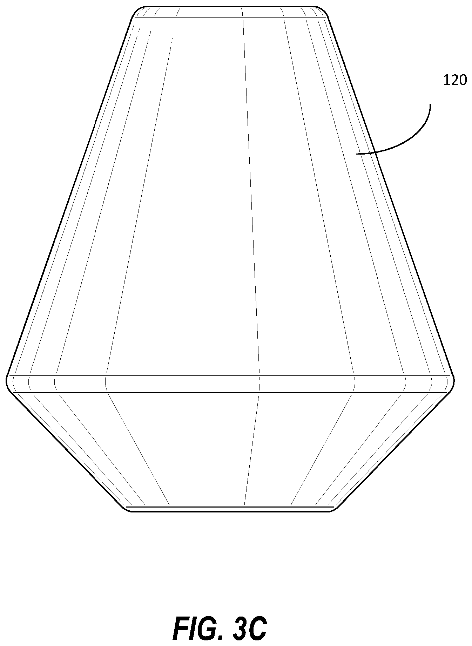

Figures 3A, 3B, and 3C illustrate various views of another example of an actuator tip 120, in accordance with various aspects of the present disclosure. In some implementations, the actuator tip 120 shown in Figures 3A, 3B, and 3C is configured for a friction-fit attachment to nozzle. In such implementations, the internal diameter of tip 120 is sized to form a secure radial interference with the nozzle or outlet tube of the actuator, allowing the tip 120 to remain attached during use while still being removable for cleaning or replacement. In other implementations, the actuator tip 120 shown in Figures 3A, 3B, and 3C includes an annular ring on an inner wall, wherein the annular ring may be coupled with a corresponding structure on the nozzle of the actuator.

As shown in the example of Figure 3A, the actuator tip 120 includes a generally tapered outer body and a centrally located lumen for receiving an actuator nozzle. The inner surface of the proximal region may include a smooth bore or be slightly contoured to enhance the surface engagement between the actuator tip and the actuator nozzle. Figure 3B illustrates an example of a rear perspective view of the actuator tip 120, in accordance with various aspects of the present disclosure. As shown in the example of Figure 3B, an inlet 302 receives the actuator nozzle. The outer body of the tip 120 may feature ergonomic shaping to improve comfort during use. Figure 3C illustrates an example of a side view of actuator tip 120. The actuator tip 120 may have a generally frustoconical geometry with a progressive taper toward both distal ends.

The examples shown in Figures 3A, 3B, and 3C do not include an integrated locking feature. Still, in some examples, the actuator tip 120 may include an internal annular ring similar to the annular ring 202 described with reference to Figures 2B and 2C. The inclusion of such a ring allows the actuator tip 120 to form a mechanical snap-fit connection with a corresponding groove or ridge on the actuator nozzle. Thus, the actuator tip 120 is not limited to a friction-fit configuration, and may be implemented using a variety of retention methods, including snap-fit, press-fit, or hybrid designs that combine mechanical and frictional retention features.

In some examples, the actuator tip 120 may be made of medical-grade silicone or another elastomeric material, and may be disposable or reusable. The internal geometry may be further configured to support a coplanar interface between the actuator nozzle and the tip interior to maintain a smooth fluid path and minimize user discomfort.

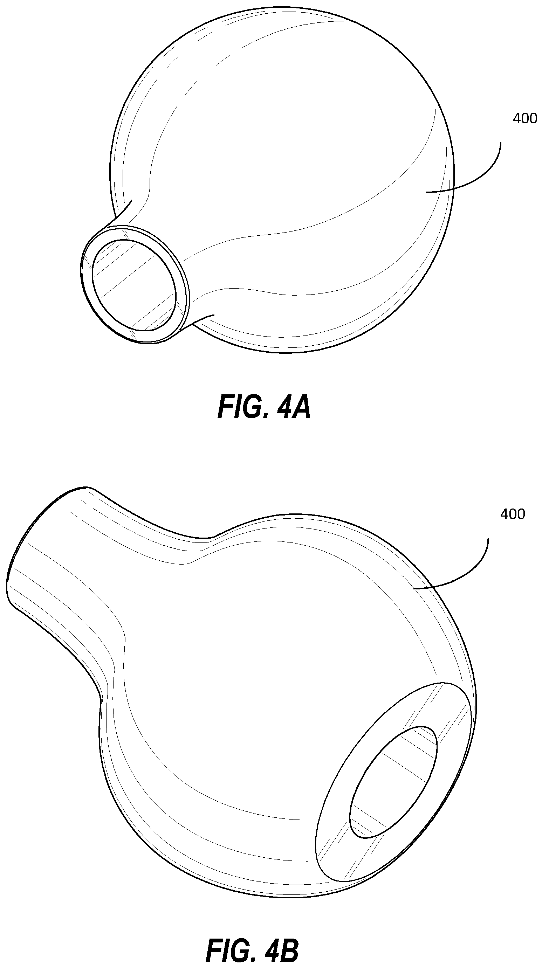

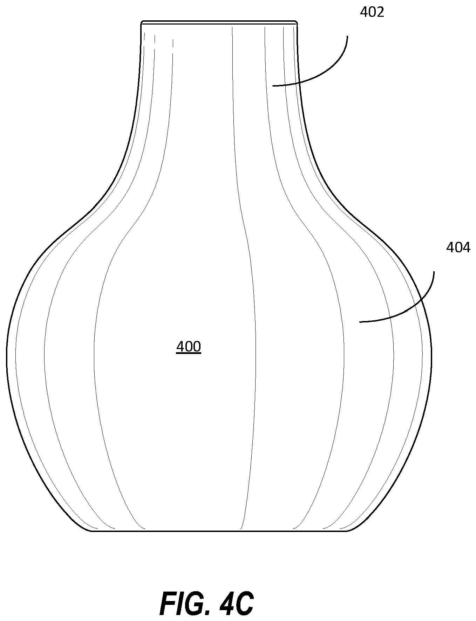

Figures 4A, 4B, and 4C illustrate another example of an actuator tip 400 for use with a nasal rinsing device, in accordance with various aspects of the present disclosure. As shown in the example of Figure 4A, the actuator tip 400 includes a bulbous or rounded outer profile with a narrowed proximal neck and a central lumen extending axially through the tip 400. The internal lumen receives the actuator nozzle, thereby allowing for delivery of saline fluid from the actuator nozzle to the user's nostril. The proximal opening of the actuator tip 400 is dimensioned to couple with a corresponding actuator nozzle using either a friction-fit configuration or a snap-fit engagement involving an annular ring.

Figure 4B illustrates an example of a rear perspective view of the actuator tip 400. The geometry of the bulbous portion provides a gentle, broad contact surface that may assist in forming a seal against the user's nostril while minimizing insertion depth. This shape may also improve the way caregivers handle aligning and inserting the tip into a child's nostril. Figure 4C illustrates a side elevation view of the actuator tip 400. As shown in the example of Figure 4C, a neck region 402 leading into the bulbous portion 404 may exhibit a smooth transition that tapers outward. This design supports a gradual interface between the nostril and the sealing surface, helping avoid sharp transitions that could cause discomfort. The internal wall of the tip 400 may be shaped to preserve a coplanar interface with the actuator nozzle, reducing internal steps or turbulence in the fluid path.

The actuator tip 400 shown in Figures 4A, 4B, and 4C may be manufactured from medical-grade silicone, thermoplastic elastomer (TPE), or another soft, biocompatible material. In some implementations, the rounded shape may also provide visual or tactile feedback to indicate proper alignment or orientation. The tip 400 may be configured to be disposable or reusable and may optionally be provided in different sizes or colors for age-based customization.

The actuator tip 400 shown in shown in Figures 4A, 4B, and 4C may attach to an actuator nozzle via a friction fit. Still, in some examples, the tip 400 may include an annular ring or similar mechanical feature to enable snap-fit retention. This flexibility in design allows for adaptation to various actuator configurations while maintaining the safety, comfort, and hygienic benefits of the system.

As discussed, various aspects of the present disclosure utilize a snap-fit design to couple a soft actuator tip with an actuator nozzle. This snap-fit engagement is configured to provide a secure mechanical connection during pressurized saline dispensing, while still allowing for user-removal of the actuator tip for hygiene or replacement. The design is particularly well-suited for pediatric applications, where both safety and comfort are desirable. While snap-fit mechanisms are generally known in other fields, such as consumer audio devices and personal care products, the structural and functional implementation in the present nasal rinse system diverges significantly from such prior art configurations.

In certain conventional designs, such as those used in earbud constructions, the snap-fit mechanism is typically embedded between two concentric walls, such as an outer wall that provides a comfortable contact surface, and an inner wall that houses the mechanical interlock. This dual-wall architecture enables the snap-fit to be implemented without altering the outer shape, feel, or profile of the contact surface. However, this construction inherently increases the material bulk and complexity of the molded component, and is specifically designed for ear canal insertion, where precision flow alignment and internal lumen smoothness are not critical.

In contrast, the actuator tip described in various aspects of the present disclosure is designed for fluid delivery through a central lumen, where both comfort and fluid dynamic performance are desirable, particularly for pediatric nasal applications. The actuator tip, in various aspects, includes a single-wall construction in which the snap-fit features, such as an annular recess or ridge, are integrated directly into the interior surface of the same wall that defines the nasal contact interface and the fluid conduit. This creates a continuous and unbroken inner wall surface extending from the actuator nozzle to the distal nasal outlet.

This approach offers a distinct advantage over conventional design. For example, unlike dual-wall designs that isolate the mechanical fit from the user-facing surface, the actuator tip provides both mechanical retention and user comfort within a unified geometry, without introducing internal steps, voids, or material overlaps that would result in discomfort or impede smooth fluid delivery. As a result, the nasal actuator tip achieves a secure and hygienic attachment to the actuator body while also maintaining a seamless, smooth interface with the user's nasal tissue and preserving laminar flow characteristics. Accordingly, a single-wall nasal actuator tip that incorporates an integrated retention feature without disrupting the internal or external comfort surfaces is distinguishable from conventional devices that use a snap-fit connection.

As discussed, the actuator tip may facilitate delivery of a fluid, such as saline solution, to a user's nasal passage. In some examples, a user may begin use of the nasal spray device by coupling the actuator tip to the actuator (e.g., the actuator nozzle). In some examples, the user aligns the actuator tip with the actuator nozzle and applies axial pressure until the snap-fit engagement is achieved. This engagement may include an annular ring on the actuator tip mating with a corresponding recess on the actuator, or vice versa. In alternative embodiments, the actuator tip may be attached via a friction-fit, whereby the inner surface of the tip is dimensioned to form an interference seal with the actuator nozzle. Upon successful attachment, the actuator tip remains in place without rotational or axial play.

Once the tip is securely attached, the user prepares to dispense the fluid, such as the saline rinse. The device is held in a single hand, with the actuator oriented such that the actuator tip can be comfortably positioned at the entrance of one nostril. The distal geometry of the actuator tip, which may be conical, bulbous, or tapered, is configured to gently seal against the outer rim of the nostril, while its insertion depth is limited by design to prevent penetration into sensitive internal nasal structures. The user may tilt their head slightly to one side, such that the opposite nostril is angled downward, allowing for optimal gravity-assisted drainage. The actuator tip is gently pressed into sealing contact with the nostril without applying force that would distort or dislodge the tip.

The user or an assistant then depresses the actuator, causing the pressurized saline solution within the canister to flow through the internal lumen of the actuator and exit through the coplanar or near-coplanar outlet of the actuator tip. The fluid is delivered in a continuous, non-atomized stream at a pressure of approximately 82.737,1 Pa and 96.526,6 Pa (i.e., approximately 12-14 psi) and a flowrate of about 1.6 mL/s-parameters selected for safe and effective rinsing for users aged two years and older. The saline solution flows through the nasal cavity and exits through the opposite nostril, carrying with it mucus, allergens, and other debris. The shape of the actuator tip and the smoothness of its interior wall help minimize resistance and user discomfort during flow.

Upon completion, the user releases the actuator, stopping the flow of solution. The actuator tip may then be removed from the nostril and either cleaned or disposed of, depending on whether the design is reusable or single-use. In reusable embodiments, the user may detach the actuator tip by pulling axially to disengage the snap-fit or friction-fit connection. The smooth, flexible geometry and material of the tip facilitate safe removal without injury or mechanical failure. The process may be repeated for the opposite nostril if desired. The actuator tip may also be sanitized between uses according to manufacturer guidelines, or replaced entirely in disposable versions.

Although many of the examples described herein feature an actuator tip formed from medical-grade silicone, the present disclosure is not limited to any particular material. The actuator tip may be formed from a wide range of biocompatible, flexible polymers, including but not limited to thermoplastic elastomers (TPE), styrene-ethylene-butylene-styrene (SEBS), natural or synthetic rubbers, polyurethane-based elastomers, or latex-free alternatives suitable for medical use. The hardness of the material may range from, for example, Shore A 50 to Shore A 70, depending on user age, comfort requirements, and durability targets. Other hardness ranges are contemplated. Similarly, while certain embodiments employ a generally conical or bulbous shape for the actuator tip, alternate geometries may include flared, oval, or asymmetrical profiles, provided they enable a seal against the nostril and limit insertion depth for safety. The outlet geometry may also be varied-circular, elliptical, or slot-shaped-to achieve desired flow characteristics.

The snap-fit engagement between the actuator and the actuator tip may be implemented using a variety of mechanically equivalent structures. For example, an annular ring on the actuator tip may engage with a groove on the actuator, or vice versa. In some embodiments, the snap-fit may be achieved using radial barbs, interference ribs, tab-and-slot configurations, bayonet couplings, or compressive flanges. These features may be continuous or segmented around the circumference of the tip or actuator. In other variants, the snap-fit may be supplemented or replaced by adhesive bonding, overmolding, or twist-lock mechanisms. These alternatives are intended to fall within the scope of the disclosure, provided they enable a secure and removable connection between the tip and the actuator body.

To support safe use in pediatric patients, the actuator tip may include features specifically tailored to younger users, such as children aged two years and older. In some examples, the actuator tip includes depth-limiting flanges or shoulders that physically prevent over-insertion into the nasal cavity. The tip geometry may also be shaped to avoid applying excessive localized pressure on the inner rim of the nostril. In certain implementations, the tip includes visual markings, color coding, or tactile indicators that guide caregivers in proper orientation, alignment, or age appropriateness. For example, actuator tips for children aged 2-4 may have smaller diameter openings and softer material formulations compared to tips intended for users aged 5 and older.

To address scenarios where the nasal passage becomes partially or fully occluded during rinsing, the actuator tip may incorporate passive or active safety features. In some examples, the actuator tip includes a pressure-relief slit, micro-valve, or vent hole that allows excess pressure to escape while maintaining directional flow. In other examples, the interior lumen may include a flow restrictor, turbulence diffuser, or swirl chamber designed to control flow velocity and reduce the risk of fluid jetting or splashback. These safety enhancements may be integrated into the tip body, molded as part of the outlet geometry, or added as insert components. These features help reduce discomfort and ensure that flow remains within the optimal pressure and velocity ranges for pediatric users.

While the system is primarily described in connection with the delivery of isotonic or hypertonic saline solutions, it may also be used to administer other nasal or upper respiratory therapeutics. These include, but are not limited to, antihistamines, decongestants, corticosteroids, homeopathic solutions, antimicrobials, or mucolytic agents. In some embodiments, the pressurized canister contains a blended solution or multi-chamber formulation, and the actuator tip is designed to maintain fluid integrity and delivery consistency. The compatibility of the actuator tip material with various fluid chemistries should be considered when selecting the composition and molding process.

The actuator tip may be manufactured using a variety of molding processes, such as injection molding, compression molding, blow molding, or overmolding onto a rigid actuator substrate. In some embodiments, the tip is co-molded with the actuator using a dual-shot process, which enables bonding between hard and soft components while preserving clean internal geometries. The outer surface of the tip may be textured, embossed, or coated to improve grip, tactile feedback, or fluid shedding. The inner surfaces of the tip and actuator may be treated with anti-microbial coatings, lubricious surface treatments, or hydrophobic layers to improve hygiene and user experience.

To ensure robust performance across a range of use conditions, the dimensional tolerances of the snap-fit and/or friction-fit interfaces may be carefully controlled. For example, the radial interference between the actuator nozzle and actuator tip may be set to achieve a pull-off force between approximately 3.5 to 4.0 pound force. The actual force required for engagement and disengagement may vary depending on material properties, wall thickness, and surface finish. In friction-fit examples, durometer tuning, draft angles, and surface roughness may be optimized to avoid unintentional detachment during use, while ensuring ease of removal for cleaning or replacement.

In some examples, the actuator tip may include flattened sections, ventilation notches, eccentric outlets, or rotationally asymmetric features for left or right nostril orientation. The base of the tip may be angled or offset relative to the flow axis to better match the anatomical contour of the nostril.

While various illustrative aspects of the actuator tip and nasal rinse apparatus have been described, the aspects of the present disclosure are not limited to the specific configurations shown. In some examples, the actuator tip may be configured with multiple outlet ports arranged in a circular or elliptical pattern to provide a diffused spray or multi-stream rinse. These configurations may be beneficial in therapeutic applications requiring broader distribution within the nasal cavity.

Further, the actuator tip may include asymmetrical geometries, such as flared or crescent-shaped outlet ends, that conform to anatomical variations or enable targeted flow direction. In some examples, the actuator tip may be rotatable or indexed to align with either the left or right nostril. Moreover, although aspects of the present disclosure have been described primarily in the context of pressurized canisters, the same actuator tip design may be adapted for use with gravity-fed, squeeze bottle, or pouch-style reservoirs.

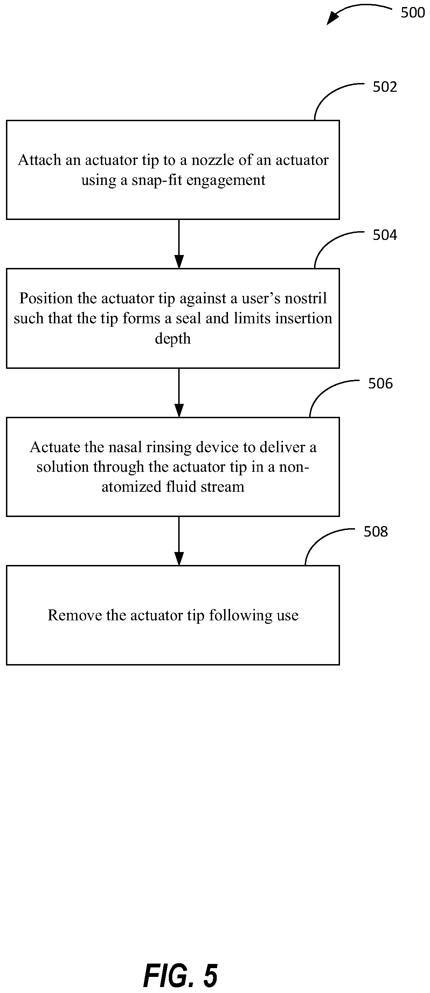

Figure 5 is a is a flow diagram illustrating a process 500 of using a nasal spray device, in accordance with various aspects of the present disclosure. The nasal spray device may be an example of the nasal spray device 100 described with reference to Figure 1. As shown in the example of Figure 5, the process 500 may be performed by a user, and begins at block 502, by attaching an actuator tip to a nozzle of an actuator using a snap-fit engagement, the actuator tip comprising a first retention feature that engages a second retention feature of the nozzle to retain the actuator tip to the nozzle during use via the snap-fit engagement. At block 504, the user positions the actuator tip against a user's nostril such that the tip forms a seal and limits insertion depth. At block 506, the user actuates the nasal rinsing device to deliver a solution through the actuator tip in a non-atomized fluid stream. At block 508, the user removes the actuator tip following use.

As used herein, the term "determining" encompasses a wide variety of actions. For example, "determining" may include calculating, computing, processing, deriving, investigating, looking up (e.g., looking up in a table, a database or another data structure), ascertaining and the like. Additionally, "determining" may include receiving (e.g., receiving information), accessing (e.g., accessing data in a memory) and the like. Furthermore, "determining" may include resolving, selecting, choosing, establishing, and the like.

As used herein, a phrase referring to "at least one of" a list of items refers to any combination of those items, including single members. As an example, "at least one of: a, b, or c" is intended to cover: a, b, c, a-b, a-c, b-c, and a-b-c.

The methods disclosed herein comprise one or more steps or actions for achieving the described method. The method steps and/or actions may be interchanged with one another without departing from the scope of the claims. In other words, unless a specific order of steps or actions is specified, the order and/or use of specific steps and/or actions may be modified without departing from the scope of the claims.

As used herein, where a quantity is specified in both the SI system and United States customary units and there is any discrepancy, the values expressed in United States customary units shall take precedence.

## Claims

1. An apparatus (100) for nasal rinsing, comprising:
a pressurized canister (102) comprising a solution and configured to deliver the solution to a user;
an actuator (104) operably coupled to the pressurized canister (102) and configured to dispense the solution in a non-atomized fluid stream upon actuation; and
an actuator tip (106, 120) formed being removably coupled to a nozzle (112) of the actuator (104), the actuator tip (106, 120) comprising a first retention feature that engages a second retention feature of the nozzle (112) to form a snap-fit that retains the actuator tip (106, 120) to the nozzle (112) during use.

2. The apparatus of claim 1, wherein the first retention feature is an annular recess and the second retention feature is an annular snap ring (108).

3. The apparatus of claim 2, wherein:
the actuator tip (106, 120) is a single-wall construction and includes an interior surface; and
the annular recess is defined on the interior surface.

4. The apparatus of any one of claims 1 to 3, wherein an outlet of the nozzle (112) and a distal end of the actuator tip (106, 120) are substantially coplanar.

5. The apparatus of any one of claims 1 to 4, wherein, the actuator tip (106, 120) comprises an elastomeric material.

6. The apparatus of any one of claims 1 to 5, wherein the actuator tip (106, 120) engages with a nostril of the user.

7. The apparatus of any one of claims 1 to 6, wherein the actuator tip (106, 120) comprises conical shape, a frustoconical shape, or a bulbous shape.

8. The apparatus of any one of claims 1 to 7, wherein the solution is a saline solution.

9. The apparatus of any one of claims 1 to 8, wherein the actuator (104) comprises a hard plastic body.

10. The apparatus of claim 1, wherein a geometry of the actuator tip (106, 120) includes limits an insertion depth into a nostril of the user.

11. A method of performing nasal irrigation using a pressurized nasal rinsing device (100), comprising:
attaching an actuator tip (106, 120) to a nozzle (112) of an actuator (104) using a snap-fit engagement, the actuator tip (106, 120) comprising a first retention feature that engages a second retention feature of the nozzle (112) to retain the actuator tip (106, 120) to the nozzle (112) during use via the snap-fit engagement;
positioning the actuator tip (106, 120) against a user's nostril such that the tip (106, 120) forms a seal and limits insertion depth;
actuating the nasal rinsing device (100) to deliver a solution through the actuator tip (106, 120) in a non-atomized fluid stream; and
removing the actuator tip (106, 120) following use.

12. The method of claim 11, wherein the actuator (104) is coupled to a pressurized canister (102) comprising the solution.

13. The method of claim 11 or 12, wherein the first retention feature is an annular recess and the second retention feature is an annular snap ring (108).

14. The method of claim 13, wherein:
the actuator tip (106, 120) is a single-wall construction and includes an interior surface; and
the annular recess is defined on the interior surface.

15. The method of any one of claims 11 to 14, further wherein the pressurized nasal rinsing device includes an apparatus (100) for nasal rinsing according to any one of claims 1 to 10.
